# DEMANDE DE BREVET EUROPEEN

(11) **EP 1 063 219 A1**
(43) Date de publication de la demande: **27.12.2000**
(21) Numéro de dépôt: 00401752.1
(22) Date de dépôt: 20.06.2000
(51) Int. Cl.: C07C 45/67, C07C 45/82, C07C 49/603

(54) **Procédé continu de fabrication de 3,5,5-triméthylcyclohexa-3-en-1-one(beta-isophorone).**

(30) Priorité: 22.06.1999 FR 9907920
(71) Demandeur: Atofina, 92800 Puteaux (FR)
(72) Inventeur: Teissier, Rémy, 69340 Francheville (FR); Martino-Gauchi, Georges, 69500 Chantilly (FR)

(57) **Abrégé**

L'invention concerne un procédé continu de fabrication de β-isophorone par isomérisation en catalyse homogène de l'α-isophorone.

Ce procédé consiste à introduire dans une zone réactionnelle l'α-isophorone et une solution d'un hydroxyde alcalin, à porter ledit milieu réactionnel à une température allant de 150°C à 216°C, à éliminer dudit milieu, simultanément, par distillation une fraction contenant une quantité pondérale de β-isophorone allant de 30 % à 90 % et, par soutirage, des produits lourds, puis à effectuer la rectification de la fraction éliminée du milieu réactionnel par distillation.

## Description

L'invention concerne un procédé continu de fabrication de 3,5,5-triméthylcyclohexa-3-en-1-one, ci-après β-isophorone par isomérisation du 3,5,5-triméthylcyclohexa-2-en-1-one, ci-après α-isophorone en phase liquide en présence d'un catalyseur homogène.

La β-isophorone est un intermédiaire de synthèse pour la fabrication de carotinoïdes, de vitamines telles que la vitamine E et de produits pharmaceutiques.

Elle intervient également directement dans des synthèses de parfums et produits naturels comme l'astaxanthine et l'acide abscisine et dérivés.

La β-isophorone est un isomère de l'α-isophorone obtenue par trimérisation de l'acétone en milieu alcalin qui se distingue de celle-ci par la position de la double liaison : la double liaison n'est plus conjuguée au carbonyle comme représenté ci-après :

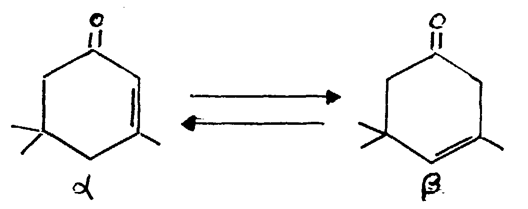

L'isomérisation de l'α-isophorone en β-isophorone est une réaction équilibrée de déconjugaison de la double liaison et du carbonyle et pour cette raison, l'équilibre thermodynamique se situe du côté de l'a-isophorone.

De nombreux procédés d'isomérisation de l'α-isophorone en β-isophorone ont été décrits mais présentent de nombreux inconvénients, tels qu'une consommation élevée de produits chimiques (catalyseurs notamment), rendements médiocres, formation de produits de condensations de l'α-isophorone (produits lourds) qui entraîne une élévation de la température du milieu réactionnel accélérant ainsi la formation de produits lourds qui déstabilisent le système.

Dans le brevet EP 832 871, exemple 7, il est décrit un procédé de préparation de β-isophorone par isomérisation catalytique de l'α-isophorone qui consiste dans un premier temps à extraire, en continu, du milieu réactionnel par distillation un mélange primaire contenant de 20 à 22 % de β-isophorone puis à isoler de ce mélange par distillation une β-isophorone avec une pureté égale à environ 98 %. On constate dans cette façon de procéder que l'on recueille dans l'évaporateur à recirculation un mélange constitué d'environ 90 % d'α-isophorone et 10 % de lourds. Compte tenu de la durée de fonctionnement (environ 15 heures) et de la quantité de produit recueilli dans ledit évaporateur (615 g), cela correspond à une production horaire de produits lourds d'environ 4 g/h.

En outre, on utilise une quantité pondérale importante de catalyseur : 0, 6 % par rapport à l'α-isophorone mis en oeuvre.

Si on considère la thermodynamique de la réaction, on constate que la température à laquelle est conduite l'isomérisation fixe la concentration en β-isophorone à l'équilibre : plus la température est haute, plus la concentration à l'équilibre en β-isophorone augmente. Comme la β-isophorone est plus volatile que l'α-isophorone, l'équilibre peut être déplacé par distillation du produit le plus volatile. En outre, la β-isophorone, par chauffage, se rétroisomérise en α-isophorone, même en absence de catalyseur : la cinétique de cette rétroisomérisation thermique est lente.

On a donc intérêt à réaliser l'isomérisation à température élevée.

L'objectif d'obtenir de la β-isophorone pure (β-isophorone / α-isophorone ≥ 99 %) ne peut être réalisé en une seule colonne à la température d'isomérisation. En effet, la séparation β-isophorone - α-isophorone exige non seulement une colonne avec de nombreux plateaux théoriques mais aussi un taux de reflux élevé. Le taux de reflux R est défini comme étant le rapport de la quantité totale de condensat obtenue en tête de distillation sur la quantité de produit extrait. L'augmentation du taux de reflux permet d'augmenter la concentration de la β-isophorone extraite jusqu'à un certain seuil. En effet, un taux de reflux élevé augmente le temps de séjour moyen de la β-isophorone dans la colonne à distiller, c'est-à-dire à température élevée, favorisant la rétrogradation thermique.

Ainsi, un taux de reflux élevé ne permet pas de produire de la β-isophorone dite pure.

D'autre part, pour conduire l'isomérisation, il faut passer par l'intermédiaire énol ou énolate qui sont aussi les intermédiaires de la réaction d'aldolisation / crotonisation qui conduit à la formation de polycondensats de l'isophorone qui constituent des produits lourds dont la formation est à proscrire puisqu'ils représentent une perte d'isophorone dans un procédé de fabrication de la β-isophorone. La formation des lourds est favorisée d'une part par la concentration en catalyseur et d'autre part par la température.

Ainsi, l'utilisation d'une catalyse hétérogène apparaît comme peu favorable car elle peut augmenter la concentration de produits lourds à la surface du catalyseur solide pouvant désactiver ledit catalyseur.

On a maintenant trouvé un procédé continu de fabrication de 3,5,5-triméthylcyclohexa-3-en-1-one (β-isophorone) par isomérisation en catalyse homogène du 3,5,5-triméthylcyclohexa-2-en-1-one (α-isophorone), obtenue par trimérisation en milieu alcalin de l'acétone, ledit procédé étant caractérisé en ce que l'on effectue les étapes suivantes :
a) on introduit en continu dans une zone réactionnelle l'α-isophorone et une solution d'un hydroxyde alcalin,
b) on porte le milieu réactionnel à une température au moins égale à 150°C, et de préférence, à une température allant de 185°C à 216°C,
c) on élimine en continu dudit milieu réactionnel, simultanément :
   1/ par distillation, à une température comprise entre 150°C et 216°C et sous une pression allant de 350 mbar à la pression atmosphérique, une fraction contenant une quantité pondérale de β-isophorone allant de 30 % à 90 % et, de préférence, allant de 50 % à 70 % ; et
   2/ par soutirage, des produits lourds de façon à ce que leur teneur soit au plus égale à 7 % en poids dans le milieu réactionnel,
d) on effectue en continu et en parallèle la rectification de la fraction distillée en c) 1/ sous pression réduite et à une température allant de 100°C à 150°C de façon à obtenir une β-isophorone d'une pureté telle que définie par le rapport β-isophorone / α-isophorone soit supérieure à 99 %, et,
e) on ramène en continu à la zone réactionnelle le pied de la rectification.

Selon la présente invention, l'hydroxyde alcalin est NaOH ou KOH. On préfère utiliser KOH. Cet hydroxyde alcalin peut être solubilisé dans de l'eau ou un alcool aliphatique de bas poids moléculaire tel que le méthanol, l'éthanol, le propanol, l'isopropanol. On utilise généralement le méthanol ou l'éthanol.

Selon la présente invention, on utilise une quantité pondérale d'hydroxyde alcalin au plus égale à 0,03 % par rapport à l'α-isophorone introduite dans la zone réactionnelle.

De préférence, on utilisera une quantité pondérale d'hydroxyde alcalin allant de 0,010 % à 0,020 %.

Le taux de reflux de la distillation de la fraction contenant la β-isophorone, paramètre réglant la concentration en β-isophorone, de cette fraction, est choisi de façon à minimiser la rétrogradation thermique, pour une pureté en β-isophorone maximale.

Selon l'invention, on extrait en continu un mélange contenant un pourcentage pondéral de lourds au plus égal à 7 %. Le reste étant un mélange d'α- et de β-isophorone. Ces lourds sont constitués essentiellement de dimères et trimères de l'isophorone et également de résidus catalytiques. Ces lourds peuvent être avantageusement introduits dans une chaîne de fabrication d'α-isophorone par catalyse basique, après la réaction de trimérisation de l'acétone et avant la neutralisation du catalyseur basique.

Le procédé continu selon la présente invention, caractérisé par un soutirage des produits lourds en continu, présente l'avantage en limitant la concentration desdits produits lourds dans le bouilleur, de stabiliser la température de l'isomérisation.

Un autre avantage du procédé de l'invention est qu'il n'est pas nécessaire d'effectuer des opérations coûteuses de traitement desdits produits lourds puisqu'ils peuvent être recyclés dans une unité de fabrication d'α-isophorone.

Le procédé de l'invention présente également l'avantage de consommer de faibles quantités de charge catalytique.

Les exemples qui suivent illustrent l'invention :

L'appareillage utilisé est constitué par :
1) un bouilleur ou réacteur d'isomérisation pouvant contenir 300 g d'α-isophorone, surmonté d'une colonne adiabatique de 1 m de long et de diamètre égal à 20 mm, contenant un garnissage structuré Sulzer EX 20 en inox 316L et une tête de colonne adiabatique à taux de reflux réglé par un Timer ;
2) une colonne de rectification identique à la colonne surmontant le bouilleur. Le mélange d'α- et de β-isophorones étant introduit en milieu de ladite colonne de rectification.

### EXEMPLE 1

Dans l'appareillage décrit ci-dessus, on introduit dans le réacteur 300 g d'α-isophorone ELF ATOCHEM S.A. contenant 0,02 % en poids de KOH(exprimé en KOH pur) préalablement dissous dans du méthanol à une concentration pondérale en KOH égale à environ 20 %.

On porte à ébullition le mélange sous une pression de 600 mbar. Le reflux débute à 195 °C et se stabilise à 196°C. La température en tête se stabilise vers 166°C. On met alors en marche le timer en réglant le taux de reflux à 80.

En même temps, on met en marche la pompe d'introduction des réactifs dans le réacteur d'isomérisation à un débit de 16,5 g/h d'α-isophorone contenant 0,004 % en poids de KOH (exprimé en KOH pur) dissous dans du méthanol et la pompe de soutirage du réacteur d'isomérisation réglée à une débit de 1,5 g/h.

Après 4 heures de fonctionnement, la température de tête se stabilise entre 175 et 176°C, et on produit 15 g/h d'un mélange β-isophorone / α-isophorone de composition moyenne 72/28.

On fait fonctionner ainsi cet appareillage durant 210 heures.

La température du réacteur d'isomérisation est stable entre 196°C et 197°C. La température de tête est constante et égale à 176-177°C. La composition et le débit du mélange extrait en tête sont constants : respectivement 72 % en β-isophorone en moyenne pour un intervalle de variation de 70 % à 75 % et 14,5 g/h en moyenne pour un intervalle de variation de 14 g/h à 15 g/h.

La productivité en β-isophorone pure est de 10 g/h en moyenne.

La composition du soutirage continu du réacteur d'isomérisation est stable et contient à partir de la huitième heure 2 % de lourds (mesuré par chromatographie en phase gazeuse) 97 % d'α- et de β-isophorones et 1 % de divers.

En 210 h, on introduit 3605 g d'α-isophorone. On produit 3036 g de mélange β-isophorone / α-isophorone 72/28 soit 2185 g d' β-isophorone. On soutire du réacteur d'isomérisation 311 g d'isophorone contenant 2 % de lourds et 1 % de divers, ce qui correspond à une perte de 0,2 % par rapport à l'isophorone introduite. Cela correspond à une production horaire de lourds de 0,03 g/h.

Le flux de tête, après un stockage dans un bac tampon est envoyé au milieu de la colonne de rectification qui travaille sous une pression de 100 mbar. La température de tête se stabilise à 110°C tandis que la température de bas de colonne se stabilise à 135°C. La fraction de tête titre une pureté en β-isophorone de 99,2 %. Le soutirage dans le bas de la colonne titre entre 6 % et 4 % de β-isophorone. Le taux de reflux est de 7. On récupère 2140 g en tête de β-isophorone à 99 %, soit 98 % de la β-isophorone introduite dans la colonne de rectification. En pied, on récupère 896 g d'α-isophorone contenant 5 % en moyenne de la β-isophorone.

Ce mélange est recyclé soit directement au réacteur d'isomérisation soit au mélangeur isophorone commerciale avec le catalyseur.

### EXEMPLE 2

Après 210 heures de fonctionnement, on modifie le réglage du taux de reflux de la colonne à distiller, dont le bouilleur est le réacteur d'isomérisation, que l'on porte à 20 et ce fonctionnement est maintenu pendant 24 heures supplémentaires.

On obtient en tête une fraction titrant 20 % en poids de β-isophorone à une débit de 52 g/h. La température en tête se stabilise à 190 °C tandis que la température du réacteur d'isomérisation reste stable à 196°C.

Le débit d'introduction de l'α-isophorone est réglé de façon à maintenir la même masse dans le réacteur d'isomérisation : débit d'introduction = 53,5 g/h en concentration 0,0005 % de KOH dans l'isophorone (dilution par 4 de la solution utilisée dans l'exemple 1). Le débit de soutirage du réacteur d'isomérisation est maintenu à 1,5 g/h et l'analyse de ce flux reste stable : 97 % environ d'isophorone, 2 % de lourds et 1 % de divers.

La productivité en β-isophorone est de 10,4 g/h en moyenne, c'est-à-dire à peu près semblable à celle de l'exemple 1.

On produit en 24 h, 240 g de β-isophorone à 99,2 % du pureté.

## Revendications

1. Procédé continu de fabrication de 3,5,5-triméthylcyclohexa-3-en-1-one(β-isophorone) par isomérisation en catalyse homogène du 3,5,5-triméthylcyclohexa-2-en-1-one(α-isophorone) obtenue par trimérisation en milieu alcalin de l'acétone, ledit procédé étant caractérisé en ce que l'on effectue les étapes suivantes :
a) on introduit en continu dans une zone réactionnelle l'α-isophorone et une solution d'un hydroxyde alcalin,
b) on porte le milieu réactionnel à une température au moins égale à 150°C,
c) on élimine en continu dudit milieu réactionnel, simultanément :
1/ par distillation, à une température comprise entre 150°C et 216°C et sous une pression allant de 350 mbar à la pression atmosphérique, une fraction contenant une quantité pondérale de β-isophorone allant de 30 % à 90 % ; et
2/ par soutirage, des produits lourds de façon à ce que leur teneur soit au plus égale à 7 % en poids dans le milieu réactionnel,
d) on effectue en continu et en parallèle la rectification de la fraction distillée en c) 1/ sous pression réduite et à une température allant de 100°C à 150°C de façon à obtenir une β-isophorone d'une pureté telle que définie par le rapport β-isophorone / α-isophorone soit supérieure à 99 %, et,
e) on ramène en continu à la zone réactionnelle le pied de la rectification.

2. Procédé selon la revendication 1, caractérisé en ce que dans l'étape b), le milieu réactionnel est porté à une température allant de 185°C à 216°C.

3. Procédé selon l'une des revendications 1 ou 2, caractérisé en ce que dans l'étape c) 1/, la fraction distillée contient une quantité pondérale de β-isophorone allant de 50 % à 70 %.

4. Procédé selon l'une quelconque des revendications 1 à 3, caractérisée en ce que l'hydroxyde alcalin est KOH.

5. Procédé selon l'une des revendications 1 ou 4, caractérisé en ce que l'hydroxyde alcalin est solubilisé dans un alcool aliphatique de bas poids moléculaire.

6. Procédé selon la revendication 5, caractérisé en ce que l'alcool aliphatique de bas poids moléculaire est le méthanol ou l'éthanol.

7. Procédé selon l'une quelconque des revendications 1 à 6, caractérisé en ce que l'on utilise une quantité pondérale d'hydroxyde alcalin au plus égale à 0,03 % par rapport à l'α-isophorone mis en oeuvre (introduite dans la zone réactionnelle).

8. Procédé selon la revendication 7, caractérisé en ce que l'on utilise une quantité pondérale d'hydroxyde alcalin allant de 0,010 % à 0,020 % par rapport à l'α-isophorone mise en oeuvre.

9. Procédé selon la revendication 1, caractérisé en ce que les produits lourds, soutirés du réacteurs d'isomérisation, étape c) 2/, sont recyclés dans une chaîne de fabrication d'α-isophorone.

10. Procédé selon la revendication 9, caractérisé en ce que les produits lourds sont introduits dans une chaîne de fabrication d'α-isophorone après la réaction de trimérisation de l'acétone et avant la neutralisation du catalyseur basique.
